(19) Europäisches Patentamt
European Patent Office
Office européen des brevets

(11) Numéro de publication : **0 382 637 B1**

(12) # FASCICULE DE BREVET EUROPEEN

(45) Date de publication du fascicule du brevet : **28.07.93 Bulletin 93/30**

(51) Int. Cl.[5] : **C07D 403/14,** A61K 31/505

(21) Numéro de dépôt : **90400337.3**

(22) Date de dépôt : **07.02.90**

(54) **Dérivés de pyrimidinyl-piperazinyl-alkyl azolès avec activité anxiolytique et/ou tranquillisante.**

(30) Priorité : **09.02.89 FR 8901700**

(43) Date de publication de la demande : **16.08.90 Bulletin 90/33**

(45) Mention de la délivrance du brevet : **28.07.93 Bulletin 93/30**

(84) Etats contractants désignés : **AT BE CH DE DK FR GB GR IT LI LU NL SE**

(56) Documents cités :
**EP-A- 0 129 128**
**US-A- 4 547 499**
**EUROPEAN JOURNAL OF MEDICINAL CHEMISTRY, vol. 22, no. 4, juillet-août 1987, pages 337-345, Elsevier, Paris, FR; G. FERRAND et al.:** "**Synthèse et activité anti-dépressive potentielle de nouvelles triazine-1,2,3 ones-4**"

(73) Titulaire : **LABORATORIOS DEL DR. ESTEVE, S.A.**
**Av. Mare de Deu de Montserrat, 221**
**E-08026 Barcelona (ES)**

(72) Inventeur : **Colombo Pinol, Augusto**
**Av. Chile, 36, 40 1a**
**E-08032 Barcelona (ES)**
Inventeur : **Frigola Constansa, Jordi**
**Av. Diagonal, 299 at. 1a**
**E-08013 Barcelona (ES)**
Inventeur : **Pares Corominas, Juan**
**Padilla, 349, 3o 3a**
**E-08025 Barcelona (ES)**

(74) Mandataire : **Ahner, Francis et al**
**CABINET REGIMBEAU, 26, avenue Kléber**
**F-75116 Paris (FR)**

EP 0 382 637 B1

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

## Description

La présente invention se rapporte à des nouveaux dérivés de 1H-azole-(ω -(4-(2-pyrimidinyl)-1-pipérazinyl)-alkyl), leur procédé de préparation, ainsi que leur application en tant que médicaments.

Les composés objets de la présente invention peuvent être utilisés dans l'industrie pharmaceutique comme intermédiaires et pour la préparation de médicaments.

On connaissait déjà des 1-4-(2-pyrimidinyl-1-pipérazinyl)-butyl-N-hétérocyclyl-diones, par exemple : Wu Y.H. et al J. Med. Chem. 1969, 12, 876, Wu Y.H. et al J. Med. Chem 1972, 15, 477; Temple D.L. et al U.S. Pat 4.456.756; Yevich J.P. et al J. Med. Chem. 1983, 26, 194, mais en revanche il n'a pas été trouvé d'exemples avec des azoles.

Nous avons maintenant découvert que les nouveaux dérivés de 1H-azole-1-(ω-(4-(2-pyrimidinyl)-1-pipérazinyl)-alkyl), qui font l'objet de la présente invention, présentent une activité biologique sur le système nerveux central, en particulier ils présentent des activités anxiolitique et tranquillisante, permettant leur emploi en thérapeutique dans le traitement de l'anxiété.

Les composés objets de la présente invention répondent à la formule générale I.

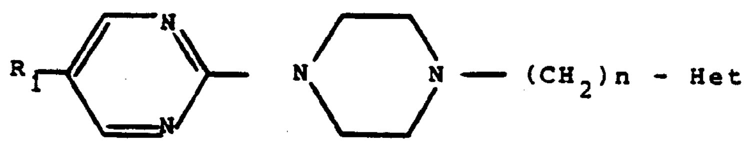

(I)

dans laquelle

$R_1$ représente un atome d'hydrogène ou un halogène,

n peut avoir les valeurs 1 à 6 et

Het représente un azole ou un de ses dérivés, choisi parmi l'imidazole, l'indazole, les tétrahydroindazoles, le pyrazole et la pyrazoline que l'on peut représenter par la formule générale II.

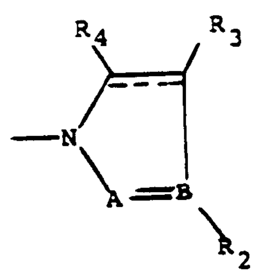

(II)

dans laquelle

A et B, toujours différents, représentent un atome de carbone ou un atome d'azote, le trait en pointillés indique la présence éventuelle d'une double liaison entre les positions 4 et 5, et

$R_2$, $R_3$ et $R_4$, identiques ou différents, pouvant également former une partie d'un autre cycle, aromatique ou non, représentent un atome d'hydrogène, un halogène, un radical alkyle inférieur, un radical nitro, un radical hydroxy, un radical oxo, un radical alcoxy, un radical cyano, un radical carboxylique, un radical carboxamido, un radical carboxylate d'alkyle, un radical phényle un radical sulfonique, un radical sulfonamido, un radical amino ou amino substitué, de formule générale III.

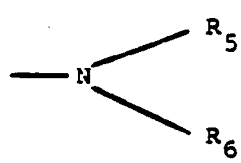

(III)

dans laquelle

$R_5$ et $R_5$, identiques ou différents, représentent un atome d'hydrogène, un radical alkyle, un radical alkényle, un radical alkylcarboxy, ou un radical alkylsulfonyle

Les nouveaux dérivés de formule genérale I peuvent être préparés, conformément à l'invention, selon l'une quelconque des méthodes suivantes.

METHODE A

Par réaction d'un composé de formule générale IV

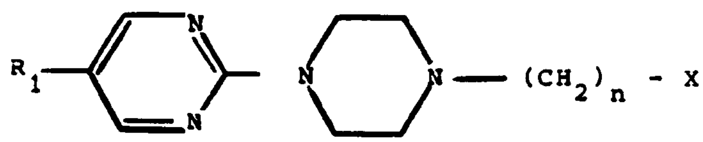

(IV)

dans laquelle

$R_1$ a les significations mentionnées précédemment et

X représente un atome d'halogène, ou un groupe partant choisi parmi le tosyloxy ou le mésyloxy, avec un composé de formule générale V

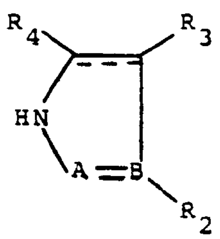

(V)

dans laquelle

A, B, $R_2$, $R_3$ et $R_4$ ont les significations mentionnées précédemment.

La réaction s'effectue en présence d'un solvant adéquat, par exemple le diméthylsulfoxyde, la diméthylformamide, un alcool, un hydrocarbure, aromatique ou non, un éther, tel le dioxanne ou l'éther diphénylique, ou un mélange de ces solvants. Cette réaction est avantageusement conduite en présence d'une base telle les hydroxydes, les carbonates ou les bicarbonates des métaux alcalins, ou bien un mélange de ces bases.

Les températures les plus adéquates varient entre la température ambiante et la température de reflux du solvant, et le temps réactionnel est compris entre 1 heure et 24 heures.

METHODE B

On suit la méthode A mais la réaction conduisant à un mélange d'isomères, on réalise en fin de réaction une séparation des composants par des méthodes physiques, telles la distillation, les cristallisations ou les méthodes chromatographiques.

METHODE C

Par réduction d'un composé de formule générale I, dans laquelle $R_1$, n et Het ont les significations mentionnées précedemment et où au moins l'un des substituants $R_2$, $R_3$ ou $R_4$, représente un groupement nitro.

Parmi les nombreux agents réducteurs susceptibles d'être utilisés pour réduire un groupe nitro jusqu'en un groupe amino, on peut citer les suivants : l'hydrogénation catalytique, en utilisant comme catalyseurs le nickel, le palladium ou le platine, l'amalgame de zinc avec de l'acide chlorhydrique, les borohydrures de métaux alcalins, etc.

La réaction s'effectue au sein d'un alcool, tel le méthanol, l'éthanol ou l'un quelconque des propanols ou des butanols, ou bien un mélange d'un alcool avec de l'eau. Les températures les plus appropriées sont comprises entre -10°C et celle de reflux du solvant, et le temps de réaction est compris entre 1 heure et 24 heures.

METHODE D

Par acylation d'un composé de formule générale I dans laquelle $R_1$, n et Het ont les significations mentionnées précédemment et où l'un des substituants $R_2$, $R_3$ ou $R_4$ représente un groupement amino, avec un halogénure d'acide ou un anhydride.

La réaction s'effectue sans solvant ou en présence d'un solvant adéquat, tel un hydrocarbure, une cétone ou un éther, et en présence d'une base, comme la pyridine ou les trialkylamines.

Les températures les plus appropriées varient entre -10°C et la température d'ébullition du solvant et le temps réactionnel est compris entre 1 heure et 24 heures.

METHODE E

Par réduction alkylative d'un composé de formule générale I, dans laquelle $R_1$, n et Het ont les significations mentionnées précédemment et où au moins l'un des substituants $R_2$, $R_3$ ou $R_4$ représente un groupement nitro, cette réduction alkylative étant réalisée avec un borohydrure de métal alcalin en présence de chlorure de nickel II et d'un composé qui possède un groupement cétone ou aldéhyde. Cette réaction s'effectue au sein d'un alcool ou d'un mélange d'alcool et d'eau.

Les températures les plus convenables varient entre -15°C et celle de reflux du solvant, et le temps réactionnel est compris entre quelques minutes et 24 heures.

METHODE F

Par réaction d'un composé de formule générale I dans laquelle $R_1$, n et Het ont les significations mentionnées précédemment mais où l'un au moins des substituants $R_1$ et $R_3$, représente un hydrogène, avec un halogène, plus particulièrement avec du chlore ou du brome. Cette réaction peut avoir lieu au sein d'un solvant approprié, tel un éther, un hydrocarbure ou un hydrocarbure halogéné, comme le tétrachlorure de carbone ou le chlorure de méthylène. Cette réaction a lieu de préférence à une température comprise entre -15°C et celle d'ébullition du solvant, et le temps de réaction est compris entre 1 heure et 24 heures.

METHODE G

Par réaction d'un composé de formule générale VI

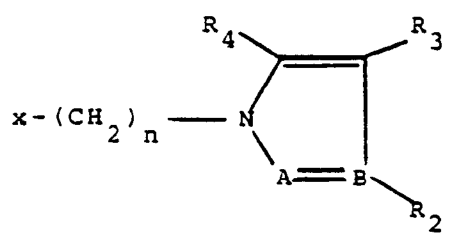

(VI)

dans laquelle

A, B, $R_2$, $R_3$, $R_4$, x et n ont les significations mentionnées précédemment, avec un composé de formule générale VII

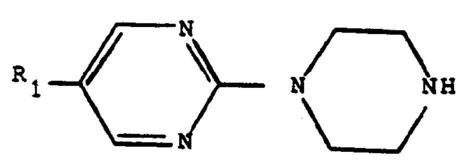

(VII)

dans laquelle

$R_1$ a les significations mentionnées précédemment.

METHODE H

Par réaction d'un composé de formule générale I avec un acide minéral ou organique dans le sein d'un solvant approprié, on obtient le sel correspondant.

Dans les exemples suivants, on indique la préparation de nouveaux dérivés selon l'invention. On décrira également quelques formes d'emploi.

Les exemples ci-après, donnés à simple titre d'illustration.

METHODE A

Exemple 1

Préparation de 1H-pyrazole-1-(4-(4-(2-pyrimidinyl)-1-pipérazinyl)-butyl)

On chauffe à reflux pendant 14 heures un mélange de 4g (13,3 mmoles) de 2-pyrimidine-1-(4-bromobutyl)-4-pipérazine, 1,02g (15 mmoles) de pyrazole et 2,76g (20 mmoles) de carbonate de potassium, dans 50 ml de diméthylformamide. On évapore sous vide, on ajoute du chloroforme, on lave à l'eau, on sèche sur du sulfate de sodium, on évapore sous vide et on obtient 3,5 g d'une huile qui est le 1H-pyrazole-1-(4-(4-(2-pyrimidinyl)-1-pipérazinyl)-butyl).

Les composés identifiés par les exemples 1 à 15, 31 à 37 et 43 à 48 sont obtenus par la même procédure et les données pour leur identification sont exposées dans les tableaux I, IV, VI et VII.

METHODE B

Exemples 25 et 26

Préparation de 1H-pyrazole-4-bromo-5-méthyl-1-(4-(4-(2-pyrimidinyl)-1-pipérazinyl)-butyl) et 1H-pyrazole-4-bromo-3-méthyl-1-(4-(4-(2-pyrimidinyl)-1-pipérazinyl)-butyl

On suit la méthode A mais avec du 4-bromo-3-(5)-méthylpyrazole.

On obtient ainsi un mélange de ces deux composants, qu'on sépare par chromatographie préparative à haute pression.

Les composés identifiés par les exemples 23 à 30, 49 et 50 sont obtenus par une méthode similaire et les données pour leur identification sont exposées dans les tableax III et VII.

METHODE C

Exemple 16

Préparation de 1H-pyrazole-4-amino-1-(4-(4-(2-pyrimidinyl)-1-pipérazinyl)-butyl)

On ajoute 10,2g (43,2 mmoles) de chlorure de nickel II hexahydrate à une solution de 7,2g (21 mmoles) de 1H-pyrazole-4-nitro-1-(4-(4-(2-pyrimidinyl)-1-pipérazinyl)-butyl) exemple n°7, dans 60 ml d'éthanol, sous forte agitation. On refroidit avec un bain de glace et on ajoute lentement 10,2g (81 mmoles) de borohydrure de sodium. On laisse sous agitation pendant 1 heure et après 1 heure à température ambiante, on additionne de l'eau, on évapore sous vide, on acidifie avec de l'acide chlorhydrique concentré, on filtre, on basifie avec de l'ammoniaque et on extrait à l'éther éthylique. On obtient ainsi 4,4g de 1H-pyrazole-4-amino-1-(4-(4-(2-pyrimidinyl)-1-pipérazinyl)-butyl) sous forme liquide.

Les données spectroscopiques pour son identification sont données dans le tableau II.

METHODE D

Exemple 17

Préparation de 1H-pyrazole-4-méthylsulfonamido-1-(4-(4-(2-pyrimidinyl)-1-pipérazinyl)-butyl)

On ajoute lentement 1,8g (16 mmoles) de chlorure de méthanesulfonyle sur une solution refroidie de 4,4g (14,6 mmoles) de 1H-pyrazole-4-amino-1-(4-(4-(2-pyrimidinyl)-1-pipérazinyl)-butyl), exemple 16, dans 30ml de pyridine. On laisse pendant une heure à 0°C, on abandonne à température ambiante pendant 4 heures, on verse sur de l'eau glacée, on extrait du chloroforme et on obtient 3,7g de 1H-pyrazole-4méthylsulfonamido-1-(4-(4-(2-pyrimidinyl)-1-pipérazinyl)-butyl), qu'on peut recristalliser dans de l'éther éthylique, avec un point de fusion de 132°C.

Les composés identifiés par les exemples 18, 19 et 38 à 42 sont obtenus par la même méthode et les données pour leur identification sont exposées dans les tableaux II et V.

METHODE E

Exemple 20

Préparation de 1H-pyrazole-4-(2-butyl)amino-1-(4-(4-(2-pyrimidinyl)-1-pipérazinyl)-butyl)

On ajoute 0,9g (24 mmoles) de borohydrure de sodium à une suspension de 2,8g (12 mmoles) de dichlorure de nickel hexahydrate, dans une solution de 2g (6 mmoles) de 1H-pyrazole-4-nitro-1-(4-(4-(2-pyrimidinyl)-1-pipérazinyl)-butyl), exemple 7, et 10 ml de méthyléthylcétone dans 50 ml d'éthanol, refroidie à 0°C. On maintient cette température pendant 30 minutes, on laisse monter jusqu'à température ambiante, on poursuit l'agitation pendant 2 heures, on évapore sous vide, on reprend avec de l'acétate d'éthyle et on obtient 1,22g de 1H-pyrazole-4-(2-butyl)amino-1-(4-(4-(2-pyrimidinyl)-1-pipérazinyl)-butyl), sous une forme liquide.

Les données spectroscopiques de ce produit sont exposées dans le tableau II.

METHODE F

Exemple 21

Préparation de 1H-pyrazole-4-bromo-1-(4-(4-(5-bromo pyrimidin-2-yl)-1-pipérazinyl)-butyl

On additionne lentement une solution de 1,84g (11,5 mmoles) de brome dans 15ml de chloroforme, à une solution de 3g (10,5 mmoles) de 1H-pyrazole-1-(4-(4-(2-pyrimidinyl)-1-pipérazinyl)-butyl) (exemple 1). On maintient l'agitation pendant 18 heures, on évapore, on reprend avec un mélange d'éther éthylique/benzène, on basifie avec de la soude 10%, on lave à l'eau, on sèche, on évapore et on obtient 3,1 g de 1H-pyrazole-4-bromo-1-(4-(4-(5-bromopyrimidin-2-yl)-1-pipérazinyl)-butyl).

Les produits des exemples 22, 25 et 26 sont obtenus par la même procédure.

Les données spectroscopiques pour l'identification de ces produits sont exposées dans les tableaux II et III.

METHODE G

Exemple 8

Préparation de 1H-pyrazole-4-chloro-1-(4-(4-(2-pyrimidinyl)-1-pipérazinyl)-butyl)

On chauffe à reflux pendant 24 heures un mélange de 3,56g (15mmoles) de N-(4-bromobutyl)-4-chloropyrazol, 2,46 g (15 mmoles) de 2-(1-pipérazinyl)-pyrimidine et 2,76g (20 mmoles) de carbonate de potassium dans 50 ml de diméthylformamide. On évapore sous vide, on ajoute du chloroforme, on lave à l'eau, on sèche sur du sulfate de sodium, on évapore sous vide et on obtient 3,2 g d'une huile qui est le 1H-pyrazole-4-chloro-1-(4-(4-(2-pyrimidinyl)-1-pipérazinyl)-butyl). Les données spectroscopiques pour l'identification de ce produit sont exposées dans le Tableau I.

METHODE H

Exemple 52

Préparation du dichlorhydrate de 1H-pyrazole-4-chloro-1-(4-(4-(2-pyrimidinyl)-1-pipérazinyl)-butyl

On dissout 5g de 1H-pyrazole-4-chloro-1-(4-(4-(2-pyrimidinyl)-1-pipérazinyl)-butyl) dans 100 ml d'alcool éthylique et on chauffe à 60°C. Moyennant de l'éthanol saturé d'acide chlrohydrique on porte la solution à pH 4,5-5. On concentre la solution résultante jusqu'à moitié volume et on laisse cristalliser à 5°C pendant 12 heures. On obtient 5,5g de cristaux de point de fusion 194-197,5°C qui correspondent au dichlorhydrate de 1H-pyrazole-4-chloro-1-(4-(4-(2-pyrimidinyl)-1-pipérazinyl)-butyl).

Le produit de l'exemple 51 est obtenu par la même procédure.

Les données spectroscopiques pour l'identification de ces produits sont exposées dans le Tableau VIII.

ACTIVITE BIOLOGIQUE

On démontre pour quelques exemples l'activite sur le système nerveux central, et plus précisément leur

activité anxiolytique et tranquillisante, au moyen du test de la réponse d'évitation conditionnée, d'après la méthode de J.S. New et cols (J.S. New, J.P. Yevich, M.S. Eison, D.P. Taylor, A.S. Eison, L.A. Riblet, C.P. Van der Maelen, D.L. Temple, J. Med. Chem. 1986, 29, 1476).

Dans ce test, on utilise des rats Wistar, mâles, de 200 grammes de poids, entraînés pour sauter une barrière dans une cage d'évitation et d'issue (shuttle box) (Letica, référence LI 910 et LI 2700) dans les 30 secondes suivantes à leur introduction dans la cage.

Les produits à activité anxiolytique ou tranquillisante suppriment la réponse d'évitation conditionnée.

Entraînement : premier jour : 11 essais, à intervalles de 3 minutes. Electrochoc sur les pattes, à 30 secondes (5 mA, 0,1 s, 10s).

Deuxième et troisième jours : 2 essais par jour, uniquement avec les rats sélectionnés [somme des ponctuations du premier jour (excepté le premier essai)> 14].

Jour de l'épreuve : Groupes formés par des rats sélectionnés. Administration orale du produit ou du véhicule 45 minutes avant le début de l'étude.

Dans le Tableau IX on résume les résultats obtenus pour quelques produits.

Compte-tenu de leurs bonnes propriétés pharmacodynamiques, les dérivés 1H-azole-(4-(4-(2-pyrimidinyl)-1-pipérazinyl)-butyl), selon l'invention, peuvent être utilisés de façon satisfaisante en thérapeutique humaine et animale, en particulier dans le traitement des troubles du système nerveux central, et plus particulièrement pour le traitement de l'anxiété ou comme tranquillisants.

En thérapeutique humaine, la dose d'administration est bien sûr fonction de la gravité de la maladie. Elle sera généralement comprise entre environ 5 et environ 100 mg/jour. Les dérivés de l'invention seront, par exemple, administrés sous forme de comprimés, de solutions ou suspensions, ou bien de gélules.

On indiquera ci-après, à titre d'exemples, deux formes galéniques particulières des dérivés objet de la présente invention.

**Exemple de formule par comprimé**

| | |
|---|---|
| Composé 1 | 5 mg |
| Lactose | 60 mg |
| Cellulose microcristalline | 25 mg |
| Povidone | 5 mg |
| Amidon prégélatinisé | 3 mg |
| Dioxyde de silice colloïdale | 1 mg |
| Stéarate de magnésium | 1 mg |
| Poids comprimé | 100 mg |

**Exemple de formule par gélule**

| | |
|---|---|
| Composé 8 | 10 mg |
| Glycéride polyoxyéthylénée | 135 mg |
| Béhénate de glycérine | 5 mg |
| Excipient : gélatine molle q.s. | 150 mg |

TABLEAU I

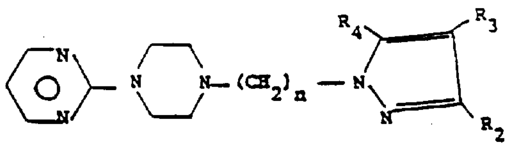

| Exemple | $R_2$ | $R_3$ | $R_4$ | n | IR $cm^{-1}$ | 1H RMN, $\delta$, $Cl_3$ CD, J= Hz |
|---|---|---|---|---|---|---|
| 1 | H | H | H | 4 | 2942, 2815, 1586, 1547, 983 | 1.50(m,2H); 1.90(m,2H); 2.40(m,6H); 3.80(m,4H); 4.12(t,2H,J=6,9); 6.20 (t,1H,J=1,6):6.40(t,1H,J=4.7); 7.42(dd,2H,J=4.7; J'=1.6); 8.25 (d.2H,J=4.7) |
| 2 | Me | H | Me | 4 | 1590, 1550, 1360, 1210, 980 | 1.58(m,2H); 1.85(m,2H); 2.20(s.3H); 2.25(s.3H); 2.44(m,6H); 3.81(m,4H); 3.97(t,2H J=7.2); 5.78(s.1H); 6.43 (t,1H,J=4,7); 8.27(d,2H,J=4.7) |
| 3 | Me | $NO_2$ | Me | 4 | 1590, 1550, 1350, 1260, 980 | 1.60(m,2H); 1.90(m,2H); 2.49(m,9H); 2.63(s.3H); 3.82(m,4H); 4.09(t,2H,J=7)6.48(t,1H,J=4.7) 8.29(d.2H.J= 4.7) |
| 4 | H | Me | H | 4 | 1590, 1550, 1500, 1360, 1260, 980 | 1.52(m,2H); 1.95(m,2H); 2.05(s,3H); 2.37(m.6H); 3.81(m,4H); 4.05(t,2H,J=6.8); 6.41(t,1H;J=4.7)7.13(S,1H) ; 7.27(s,1H); 8.25(d.2H, J=4.7) |
| 5 | H | -CH=CH-CH=CH- | | 4 | 2930, 1590, 1550, 1500, 1360, 1310, 1260, 980 | 1.51(m,2H); 1.98(m,2H); 2.36(m,6H); 3.77(m.4H); 4.39(t,2H,J=6.9); 6.40 (t,1H,J=4.7); 7.0-7.7 (m,4H); 7.95 (s,1H); 8.25(d,2H,J=4.7) |
| 6 | Me | Br | Me | 4 | 2930, 1590, 1550, 1500, 1360, 1310, 1260, 980 | 1.55(m,2H); 1.81(m,2H); 2.18(s,3H); 2.20(s.3H); 2.38(m,4H); 3.80(m,4H); 3.99(t,2H,J=6.9); 6.42(t,1H,J=4.7); 8.25(d,2H,J=4.7) |
| 7 | H | $NO_2$ | H | 4 | 1584, 1524, 1480, 1444, 1406, 1359, 1305, 819 | 1.5(m,2H); 1.93(m,2H); 2.38(m,6H); 3.76(m.4H); 4.15(t,2H,J=6.7); 6.42 (t,1H,J=4.7); 8.01(s,1H)8.12(s.1H); 8.24(d,2H,J=4.7) |
| 8 | H | Cl | H | 4 | 2843, 1586, 1547, 1358, 983 | 1.52(m,2H); 1.90(m,2H); 2.43(m,6H); 3.80(m.4H); 4.0(t,2H,J=6.8); 6.44 (t,1H,J=4.7); 7.35(s,1H) 7.39 (s.1H); 8.25(d,2H,J=4.7) |
| 9 | H | EtOOC- | H | 4 | 1715, 1586, 1222, 983 | 1.34(t,3H,J=7.1); 1.54(m,2H); 1.90 (m.2H); 2.46 (m,6H); 3.81(m,4H);4.25 (m,4H); 6.47(t,1H,J=4.7); 7.90 (s.2H); 8.29(d,2H,J=4.7) |

TABLEAU I ( suite)

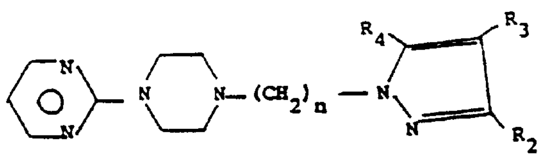

| Exemple | $R_2$ | $R_3$ | $R_4$ | n | IR $cm^{-1}$ | 1H RMN, $\delta$ , $Cl_3$ CD, J= Hz |
|---|---|---|---|---|---|---|
| 10 | H | Me | =0 | 4 | 1587, 1548, 1447, 1360, 1260, 984 | 1.72(m,4H) ; 1.90(s,3H) ; 2.48(m,6H) ; 3.80(m,4H) ; 4.18(t,2H,J=7,0) ; 6.43 (t,1H,J=4,7) ; 7.09(s,1H) ; 8.26(d,2H, J=4.7) |
| 11 | Me | H | Ph | 4 | 1586, 1547, 1360, 983 | 1.54(m,2H) ; 1.85(m,2H) ; 2.28(s.3H) ; 2.45(m.6H) ; 3.81(m,4H) ; 4.07(t,2H, J=7) ; 6.28(s,1H) ; 6.43(t,1H,J= 4,7) ; 7.33(m,4H) ; 7.75(m,2H) ; 8.26(d,2H, J=4.7) |
| 12 | H | Br | H | 4 | 1586, 1547, 1360, 984 | 1.52(m,2H) ; 1.89(m,2H) ; 2.44(m.6H) ; 3.62(m.4H) ; 4.11(t,2H,J=6,7) ; 6.46 (t,1H,J=4.6) ; 7.42(s,1H) ;7.45 (s,1H) ; 8.29(d,2H,J=4.6) |
| 13 | H | C≡N | H | 4 | 3076, 2231, 1587, 1551, 1258, 982 | 1.54(m,2H) ; 1.96(m,2H) ; 2.40(m.6H) ; 3.81(m.4H) ; 4.20(t,2H,J=6,9) ; 6.48 (t,1H,J=4.7) ; 7.80(s,1H) ;7.83 (s,1H) ; 8.29(d,2H,J=4.7) |
| 14 | H | F | H | 4 | 2944, 1584, 1546, 1507, 1359, 1260, 983 | 1.45(m,2H) ; 1.96(m,2H) ; 2.36(m.6H) ; 3.77(m.4H) ; 4.0(t,2H,J=6,9) ; 6.47 (t,1H,J=4.7) ; 7.27(m,2H,J=4.8) ; 8.29(d,2H,J=4,8) |
| 15 | H | Me-O- | H | 4 | 2940, 1585, 1547, 1470, 1359, 1122, 983 | 1.54(m,2H) ; 1.89(m,2H) ; 2.42(m.6H) ; 3.77(m.7H) ; 4.06(m,2H) ; 6,42 (t,1H, J=4.7) ; 7.02(s,1H)7.26(m,2H) ; 8.25 (d,2H,J=4,6) |

TABLEAU II

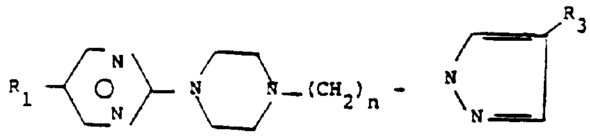

| Exemple | $R_3$ | $R_1$ | P.F. | n | IR | 1H RMN, δ, $Cl_3$ CD, J= Hz |
|---|---|---|---|---|---|---|
| 16 | $H_2N$- | H | huile | 4 | 1586, 1548 1360, 984 | 1.50(m,2H); 1.85(m,2H); 2.43(m,6H); 3.4(élargie 2H); 3.8(m,6H); 4.0 (t,2H,J=6.4); 6.46(t,1H,J=4.7); 6.98(s,1H); 7.10(s,1H); 8.27(d,2H, J=4.7) |
| 17 | Me-$SO_2$-NH- | H | 132ºC | 4 | 1582, 1482 1360, 1150 983 | 1.58(m,2H); 1.93(m,2H); 2.45 (m,6H); 2.94(s,3H); 3.8(m,4H);4.11 (t,2H,J=6.9); 6.45(t,1H,J=4.7); 7.4(s,1H); 7.5(s,1H) 8.28(d,2H, J=4.7) |
| 18 | Ph-CO-NH- | H | 134-6ºC | 4 | 1646, 1586 1542, 1369 | 1.55(m,2H); 1.79(s,3H); 1.88 (m,2H); 2.42(m,6H); 3.80(m,4H); 4.13(t,2H,J=6.8); 6.51(t,1H,J=4.7); 7.49(m,4H); 7.83(m,2H); 8.0 (s,1H); 8.11(s.1H); 8.28(d,2H,J=4.7) |
| 19 | Me-CO-NH- | H | 80-2ºC | 4 | 1650, 1586 1454, 1364 1261, 983 | 1.50(m,2H); 1.88(m,2H); 2.11 (s,3H); 2.43(m,6H); 3.79(m,4H); 4.08(t,2H,J=6.8); 6.47(t,1H,J=4.7); 7.36(s,1H)7.93(s,1H); 8.28 (d,2H,J=4.6); 9.25(s,1H) |
| 20 | Me<br>CH-NH-<br>Et | H | huile | 4 | 2960, 1585 1547, 1359 1260, 983 | 1.00(t,3H,J=7.0); 1.19(d,3H,J=6.3); 1.6(m,4H); 1.90(m,2H); 2.50 (m,6H); 3.0(m,3H); 3.9(m,4H); 4.1 (t,2H,J=6.8); 6.52(t,1H,J=4.7); 6.99(s.1H); 7.17(s,1H); 3.37 (d,2H,J=4.7) |
| 21 | Br | Br | 84.6 | 4 | 2952, 1583 1526, 1365 1311, 950 | 1.57(m,2H); 1.90(m,2H); 2.45(m,6H); 3.80(t,4H,J=6.8); 7.44(d,2H, J=4); 8.29(s,2H) |
| 22 | Cl | Br | 85-6ºC | 4 | 1585, 1525 1495, 1364 | 1.50(m,2H); 1.86(m,2H); 2.40(m,6H); 3.76(m,4H); 4.08(m,2H); 7.4 (t,2H,J=6.9); 8.25(s,2H) |

TABLEAU III

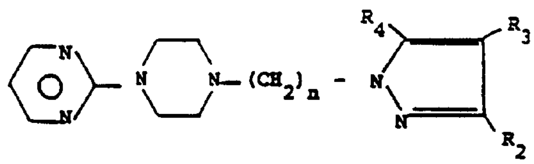

| Exemple | $R_2$ | $R_3$ | $R_4$ | n | IR $cm^{-1}$ | 1H RMN, $\delta$ , $Cl_3$ CD, J= Hz |
|---|---|---|---|---|---|---|
| 23 | H | H | Me | 4 | 1585, 1550, 1500, 1450, 1360, 980 | 1.50(m,2H); 1.80(m,2H); 2.29(s,3H); 2.39(m,6H); 3.82(m,4H); 4.04(t,2H, J=6,9); 5.97(s,1H); 6.40(t,1H,J=4.7); 7,34(d,1H,J=2.1); 8.24(d,2H, J=4.7) |
| 24 | Me | H | H | 4 | 1585, 1550, 1500, 1450, 1360, 980 | 1.52(m,2H); 1.81(m,2H); 2.25(s.3H); 2.44(m.6H); 3.81(m,4H); 4.03(t,2H, 5.95(s,1H) 6.42(t,1H,J=4.7); 7.23 (d,1H,J=2.1); 8.27(d,2H,J=4.7) |
| 25 | H | Br | Me | 4 | 1590, 1550, 1500, 1450, 1360, 1260, 980 | 1.52(m,2H); 1.83(m,2H); 2.26(s.3H); 2.45(m.6H); 3.80(m,4H); 6.45(t,1H, J=4.7); 7.38(d,1H,J=1.8); 8.27 (d,2H,J=4.7) |
| 26 | Me | Br | H | 4 | 1590, 1550, 1500, 1450, 1360, 1260, 980 | 1.53(m,2H); 1.84(m,2H); 2.22(s.3H); 2.45(m.6H); 3.80(m,4H); 6,46(t,1H, J=4.7); 7.31(d,1H,J=1.7); 8.29 (d,2H,J=4.7) |
| 27 | H | $-(CH_2)_4-$ | | 4 | 2930, 1590, 1550, 1500, 1450, 1360, 1310, 1260, 980 | 1.70(m,8H); 2.45(m,10H); 3.8(m.4H); 4.04(t,2H,J=6,9); 6.43(t,1H,J=4.7); 7.23(d,1H,J=1.8); 8.26(d,2H,J=4,7) |
| 28 | $-(CH_2)_4-$ | | H | 4 | 2930, 1590, 1550, 1500, 1450, 1360, 1310, 1260, 980 | 1.70(m,8H); 2.45(m,10H); 3.8(m.4H); 3.97(t,2H,J=6,9); 6.45(t,1H,J=4.7); 7.05(d,1H,J=1.8); 8.27 (d,2H,J=4.7) |
| 29 | H | Ph | Me | 4 | 1590, 1550, 1500, 1450, 1360, 1310, 1260, 980 | 1.50(m,2H); 1.90(m,2H); 2.39(s.3H); 2.50(m.6H); 3.80(m,4H); 4,1(t,2H, J=6.9); 6.44(t,1H,J=4.7); 7.35 (m,6H); 8.27(d,2H,J=4.7) |
| 30 | Me | Ph | H | 4 | 1590, 1550, 1500, 1450, 1360, 1310, 1260, 980 | 1.50(m,2H); 1.90(m,2H); 2.40(s.3H); 2.51(m.6H); 3.81(m,4H); 4,09(t,2H, J=6.9); 6.44(t,1H,J=4.7); 7.34 (m,6H); 8.28(d,2H,J=4.7) |

Tableau IV

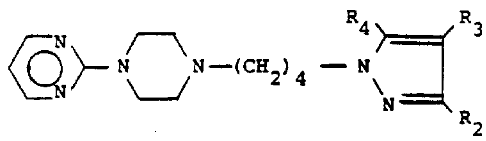

| Exemple | $R_2$ | $R_3$ | $R_4$ | IR $cm^{-1}$ | 1H RMN, δ, $Cl_3$ CD, J= Hz |
|---|---|---|---|---|---|
| 31 | Cl | F | H | 2944, 1585, 1547, 1507, 1360, 1260, 984 | 1.52(m,2H); 1.90(m,2H); 2.40(m,6H); 3.80(m,4H); 4.0(t,2H,J=4,8); 6.45 (t,1H,J=4,7); 7.30(d,1H,J=4,8); 8.29 d,2H,J=4,8) |
| 32 | Cl | Me–O | H | 2940, 1586, 1470, 1360, 1121, 983 | 1.53(m,2H); 1.90(m,2H); 2.4(m,6H); 3.8(m.7H); 4.0(m,2H); 6.4(t,1H, J=4,8); 7.0(s,1H); 7.25 (s,1H); 8.2(d,2H,J=4,8) |
| 33 | H | 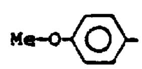 | H | 2390, 1589, 1545, 1495, 1360, 1247, 983, 835, 799 | 1.62(m,2H); 1.88(m,2H); 2.45(m.6H); 3.81(m,7H); 4.16(t,2H,J=6,8); 6.46 (t,1H,J=4.7); 6.9(d,2H,J=4,4); 7.4 (d,2H,J=4,4); 7,55(s,1H); 7.7 (s,1H) 8.28(d,2H,J=2,4) |
| 34 | H | 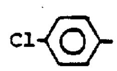 | H | 2946, 1586, 1549, 1485, 1395, 1257, 982, 951, 830 | 1.6(m,2H); 1.9(m,2H); 2.46(m,6H); 3.8(m.4H); 4.16(t,2H,J=6,8); 6.4 (t,1H,J=4.7); 7.36(d,4H,J=1,3); 7,7(d,2H,J=6,2); 8.28(d,2H,J=2,3) |
| 35 | H | 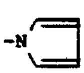 | H | 2943, 1586, 1487, 1359, 1260, 984, 726 | 1.55(m,2H); 1.80(m,2H); 2.45(m,6H); 3.81(t,4H,J=5); 4.12(t,2H,J=7); 6.25(2H,t,J=2); 6.44(1H,t,J=4,7); 6.84(m,2H); 7.5(d,2H,J=5); 8.27 (d,2H,J=4,7) |
| 36 | H | 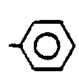 | H | 2942, 1585, 1493, 1446, 1359, 1258, 983, 760 | 1.6(m,2H); 1.9(m,2H); 2.5(m,4H); 3.8(m,6H); 4.2(t,2H,J=6,8) 6.7(t,1H, J=4,7); 7.2-7.7(abs. compl. 5H); 8.0 (s,1H); 8.2(s,1H); 8,4(d,2H,J=2,3) |
| 37 | 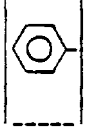 | H | 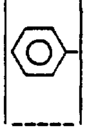 | 2942, 1585, 1547, 1485, 1359, 1260, 983, 763, 697 | 1.6(m,2H); 1.9(m,2H); 2.35(m,6H); 3.8(m,4H); 4.2(t,2H,J=6.8); 6.4 (t,1H,J=4,7); 6.6(s,1H); 7.2-7.4 (abs. compl. 8H); 7.8(m,2H); 8,25 (d,2H,J=2,4) |

Tableau V

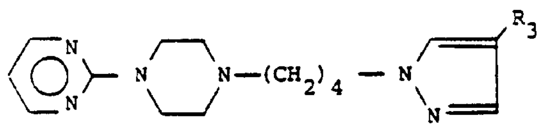

| Exemple | $R_3$ | IR $cm^{-1}$ | 1H RMN, δ, $Cl_3$ CD, J= Hz |
|---|---|---|---|
| 38 | 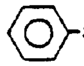 $-SO_2-NH-$ | 2931, 1584, 1548, 1490, 1358, 1167, 983 | 1.45(m,2H); 1.85(m,2H);2.40(m,6H); 3.80(m,4H); 4.0(t,2H,J=6,7); 6.47 (t,1H,J=4.6); 7.0(s,1H);7.5(m,6H); 8.3(d,2H,J=4.6) |
| 39 | Me- 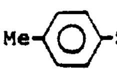 $-SO_2-NH$ | 2943, 1585, 1548, 1446, 1360, 1161, 984 | 1.5(m,2H); 1.85(m,2H); 2.28(m,9H); 3.8(m,4H); 4.0(m,2H); 6.45(t,1H, J=4,7); 7-7.65 (m,6H); 8.27(d,2H, J=4,7) |
| 40 | n-Bu-$SO_2$-NH- | 2941, 1586, 1548, 1448, 1360, 1146, 984, 755 | 0.91(t,3H,J=6,8); 1.45(m,4H); 1.85 (m,4H); 2.40(m,6H); 3.0(m,2H); 3.80(m,4H); 4.11(t,2H,J=6,5); 6.5(t,1H,J=4,7); 7.4(m,2H); 7.5 (s,1H); 8.3(d,2H,J=4.7) |
| 41 | n-Pr-$SO_2$-NH- | 2940, 1586, 1548, 1447, 1360, 1146, 984, 755 | 1.0(t,3H,J=7,1); 1.55(m,2H); 1.9 (m,4H); 2.45(m,6H); 3.0(t,2H,J= 7.4); 3.8(m,4H); 4.1(t,2H,J=6,4); 6.46(t,1H,J=4,7); 7.35(m,2H); 7.5 (s,1H); 8.3(d,2H,J=4,7) |
| 42 | Et-$SO_2$-NH- | 2943, 1586, 1548, 1447, 1360, 1146, 984, 754 | 1.36(m,5H); 1.9(m,2H); 2.45(m,6H); 3.0(m,2H); 3.6(m,4H); 4.1(t,2H, J=6.4); 6.45(t,1H,J=4,7); 7.39 (s,1H); 7.51(s,1H);8.3(d,2H,J=4,7) |

Tableau VI

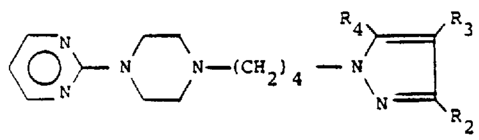

| Exemple | $R_2$ | $R_3$ | $R_4$ | IR $cm^{-1}$ | 1H RMN, δ, $Cl_3$ CD, J= Hz |
|---|---|---|---|---|---|
| 43 | Me | $-SO_2-N-Me_2$ | Me | 2939, 1586, 1547, 1448, 1360, 1290, 983, 951, 788 | 1.7(m,4H); 2.3-3.0(abs. compl. 18H); 3.8(m,4H); 4.0(t,2H,J=6,8); 6.5 (t,1H,J=4,7); 8.2(d,2H,J=2,35) |
| 44 | H | $-SO_2-N-Me_2$ p.f. 100-2ºC | H | 3135, 2943, 1586, 1512, 1357, 1328, 1156, 982, 728 | 1.6(m,2H); 1.9(m,2H); 2.3-2.7(abs. compl. 13H); 3.8(m,4H); 4.2(t,2H, J=6,8); 6.4(t,1H,J=4,7); 7.75(d,1H, J=4,4); 8.28(d,2H,J=2,4) |
| 45 | H | $-SO_3-H$ p.f. 235ºC | H | 3330, 1590, 1556, 1449, 1220, 1178, 1049, 971, 656 | 1.95(m,2H); 3.3(m,6H); 4.0(s,5H); 4.27(t,2H,J=6,1); 6.8(t,1H,J=4,8); 7.8(s,1H); 8.0(s,1H); 8.43 (d,2H, J=2,4) |

Tableau VII

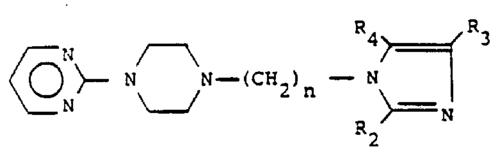

| Exemple | $R_2$ | $R_3$ | $R_4$ | n | IR $cm^{-1}$ | 1H RMN, δ, $Cl_3$ CD, J= Hz |
|---|---|---|---|---|---|---|
| 46 | H | H | H | 4 | 2940, 1585, 1500, 1360, 1260, 975, | 1.6(m,2H); 1.8(m,2H); 2.5(m,6H); 3.80(m,6H); 6.5(t,1H,J=4,7); 6.9 (s,1H); 7.1(s,1H); 7.5(s,1H); 8.4 (d,2H,J=4,7) |
| 47 | Me | H | H | 4 | 2941, 1586, 1547, 1499, 1359, 1259 983 | 1.72(m,4H); 2.37(s,3H); 2.44(m.6H); 3.80(m.6H); 6.45(t,1H,J=4,7); 6.85 d,2H,J=4,5); 8.27(d,2H,J=4,7) |
| 48 | H | Cl | Cl | 4 | 2946, 1584, 1543, 1492, 1359, 1254, 983, 797 | 1.4-2.1(abs. compl. 4H); 2.46(m,6H); 3.86(m,6H); 6.47(t,1H,J=4,7); 7.38 (s,1H); 8.29 (d,2H,J=4,7) |
| 49 | H | Me | H | 4 | 2942, 1585, 1548, 1447, 1359, 1260, 984, 735 | 1.4-2.0(abs. compl. 4H); 2.21(s,3H); 2.45(m.6H); 3.82(m,6H); 6.47(t,1H, J=4,7); 6.62(s,1H); 7,35(s,1H); 8,28 (d,2H,J=4,7) |
| 50 | H | H | Me | 4 | 2942, 1585, 1548, 1446, 1359, 1260, 984, 736 | 1.4-2.0(abs. compl. 4H); 2.20(s,3H); 2.45(m.6H); 3.82(m,6H); 6,47(t,1H, J=4,7); 6.79(s,1H); 7,40(s,1H); 8,28 (d,2H,J=4,7) |

Tableau VIII

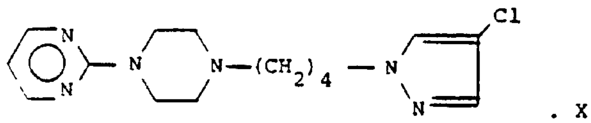

| Exemple | X | P.F. ºC | IR $cm^{-1}$ |
|---|---|---|---|
| 51 | 1 HCl | 156-8 | 3490, 1592, 1556, 1481, 1438, 1386, 970 |
| 52 | 2 $HCl.H_2O$ | 194-197.5 | 3429, 2688, 1636, 1620, 1346, 1218, 971 |

TABLEAU IX

| Exemple | Activité % | D.E$_{50}$ (mg/kg) |
|---|---|---|
| 1 | 98 | 26.2 |
| 2 | 50 | 80.0 |
| 3 | 61 | 58.3 |
| 4 | 98 | < 20.0 |
| 5 | 45 | 80.0 |
| 6 | 51 | 80.0 |
| 7 | 98 | 6.2 |
| 8 | 95 | 17.9 |
| 9 | 14 | - |
| 10 | 94 | 25.7 |
| 11 | 24 | - |
| 12 | 95 | 28.9 |
| 13 | 100 | - |
| 14 | 85 | - |
| 16 | 79 | 27.7 |

Tableau IX (suite)

| Exemple | Activité % | $D.E_{50}$ (mg/kg) |
|---|---|---|
| 17 | 64 | 40.0 |
| 18 | 62 | 65.9 |
| 19 | 89 | 28.8 |
| 20 | 80 | 29.7 |
| 21 | 59 | 76.8 |
| 22 | 48 | 51.7 |
| 23 | 47 | - |
| 25 | 53 | 60.6 |
| 26 | 59 | 58.3 |
| 27 | 97 | 19.5 |
| 28 | 98 | 18.9 |
| 29 | 75 | 32.2 |
| 30 | 78 | 31.9 |
| 31 | 85 | 53.9 |
| 33 | 60 | 22.5 |

Tableau IX (suite)

| Exemple | Activité % | D.$E_{50}$ (mg/kg) |
|---|---|---|
| 34 | 90 | 15.7 |
| 35 | 100 | 5 |
| 36 | 100 | 27.4 |
| 37 | 31 | 122 |
| 38 | 46 | 85 |
| 39 | 37 | 102 |
| 40 | 57 | 73 |
| 41 | 57 | 73 |
| 42 | 77 | 29.3 |
| 43 | 45 | 82 |
| 44 | 63 | 72 |
| 45 | 15 | - |
| 46 | 35 | 93 |
| 47 | 35 | 107 |
| 48 | 99 | - |
| 49 | 50 | 80 |
| 50 | 52 | 75 |
| 51 | 100 | 14.5 |
| 52 | 99 | 4.9 |
| Buspirona | 99 | 17.2 |
| Ipsapirona | 98 | 26.1 |

## Revendications

1. Composés hétérocycliques caractérisés en ce qu'ils répondent à la formule générale I

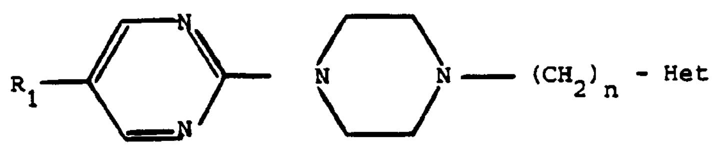

(I)

dans laquelle

$R_1$ représente un atome d'hydrogène ou un halogène,

n peut avoir les valeurs 1 à 6 et

Het représente un azole ou un de ses dérivés, choisi parmi l'imidazole, l'indazole, les tétrahydroindazoles, le pyrazole et la pyrazoline que l'on peut représenter par la formule générale II.

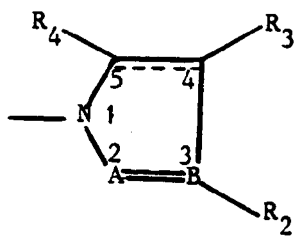

(II)

dans laquelle

A et B, toujours différents, représentent un atome de carbone ou un atome d'azote, le trait en pointillés indique la présence éventuelle d'une double liaison entre les positions 4 et 5, et

$R_2$, $R_3$ et $R_4$, identiques ou différents, pouvant également former une partie d'un autre cycle, aromatique ou non, représentent un atome d'hydrogène, un halogène, un radical alkyle inférieur, un radical nitro, un radical hydroxy, un radical oxo, un radical alcoxy, un radical cyano, un radical carboxylique, un radical carboxamido, un radical carboxylate d'alkyle, un radical phényle, , un radical sulfonique, un radical sulfonamido, un radical amino ou amino substitué, de formule générale III.

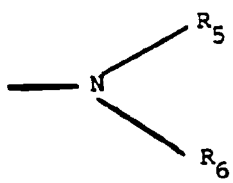

(III)

dans laquelle

$R_5$ et $R_6$, identiques ou différents, représentent un atome d'hydrogène, un radical alkyle, un radical alkényle, un radical alkylcarboxy, ou un radical alkylsulfonyle.

2. Les composés répondant à la formule générale I selon la revendication 1, sélectionnés parmi le groupe suivant :

1 - 1H-pyrazole-1-(4-(4-(2-pyrimidinyl)-1-pipérazinyl)-butyl),
2 - 1H-pyrazole-3,5-diméthyl-1-(4-(4-(2-pyrimidinyl)-1-pipérazinyl)-butyl),
3 - 1H-pyrazole-3,5-diméthyl-4-nitro-1-(4-(4-(2-pyrimidinyl)-1-pipérazinyl-butyl),
4 - 1H-pyrazole-4-méthyl-1-(4-(4-(2-pyrimidinyl)-1-pipérazinyl)-butyl),
5 - 1H-indazole-1-(4-(4-(2-pyrimidinyl)-1-pipérazinyl)-butyl),
6 - 1H-pyrazole-3,5-diméthyl-4-bromo-1-(4-(4-(2-pyrimidinyl)-1-pipérazinyl)-butyl),
7 - 1H-pyrazole-4-nitro-1-(4-(4-(2-pyrimidinyl)-1-pipérazinyl)-butyl),
8 - 1H-pyrazole-4-chloro-1-(4-(4-(2-pyrimidinyl)-1-pipérazinyl)-butyl),
9 - 1H-pyrazole-4-carboxylate d'éthyle-1-(4-(4-(2-pyrimidinyl)-1-pipérazinyl)-butyl),
10 - 1H-pyrazoline-4-méthyl-5-one-1-(4-(4-(2-pyrimidinyl)-1-pipérazinyl)-butyl)
11 - 1H-pyrazole-3-méthyl-5-phényl-1-(4-(4-(2-pyrimidinyl)-1-pipérazinyl)-butyl),
12 - 1H-pyrazole-4-bromo-1-(4-(4-(2-pyrimidinyl)-1-pipérazinyl)-butyl),
13 - 1H-pyrazole-4-cyano-1-(4-(4-(2-pyrimidinyl)-1-pipérazinyl)-butyl),
14 - 1H-pyrazole-4-fluoro-1-(4-(4-(2-pyrimidinyl)-1-pipérazinyl)-butyl),

15 - 1H-pyrazole-4-méthoxy-1-(4-(4-(2-pyrimidinyl)-1-pipérazinyl)-butyl),
16 - 1H-pyrazole-4-amino-1-(4-(4-(2-pyrimidinyl)-1-pipérazinyl)-butyl),
17 - 1H-pyrazole-4-méthylsulfonamido-1-(4-(4-(2-pyrimidinyl)-1-pipérazinyl)-butyl),
19 - 1H-pyrazole-4-acétamido-1-(4-(4-(2-pyrimidinyl)-1-pipérazinyl)-butyl),
20 - 1H-pyrazole-4-(2-butyl)amino-1-(4-(4-(2-pyrimidinyl)-1-pipérazinyl)-butyl),
21 - 1H-pyrazole-4-bromo-1-(4-(4-(5-bromopyrimidin-2-yl)-1-pipérazinyl)-butyl),
22 - 1H-pyrazole-4-bromo-1-(4-(4-(5-chloropyrimidin-2-yl)-1-pipérazinyl)-butyl),
23 - 1H-pyrazole-5-méthyl-1-(4-(4-(2-pyrimidinyl)-1-pipérazinyl)-butyl),
24 - 1H-pyrazole-3-méthyl-1-(4-(4-(2-pyrimidinyl)-1-pipérazinyl)-butyl),
25 - 1H-pyrazole-4-bromo-5-méthyl-1-(4-(4-(2-pyrimidinyl)-1-pipérazinyl)-butyl),
26 - 1H-pyrazole-4-bromo-3-méthyl-1-(4-(4-(2-pyrimidinyl)-1-pipérazinyl)-butyl)
27 - 1H-4,5,6,7-tétrahydroindazole-1-(4-(4-(2-pyrimidinyl)-1-pipérazinyl)-butyl),
28 - 2H-3,4,5,6-tétrahydroindazole-2-(4-(4-(2-pyrimidinyl)-1-pipérazinyl)-butyl),
29 - 1H-pyrazole-5-méthyl-4-phényl-1-(4-(4-(2-pyrimidinyl)-1-pipérazinyl)-butyl),
30 - 1H-pyrazole-3-méthyl-4-phényl-1-(4-(4-(2-pyrimidinyl)-1-pipérazinyl)-butyl),
31 - 1H-pyrazole-3-chloro-4-fluoro-1-(4-(4-(2-pyrimidinyl)-1-pipérazinyl)-butyl),
32 - 1H-pyrazole-3-chloro-4-méthoxy-1-(4-(4-(2-pyrimidinyl)-1-pipérazinyl)-butyl),
36 - 1H-pyrazole-4-phényl-1-(4-(4-(2-pyrimidinyl)-1-pipérazinyl)-butyl),
37 - 1H-pyrazole-3,5-diphényl-1-(4-(4-(2-pyrimidinyl)-1-pipérazinyl)-butyl),
40 - 1H-pyrazole-4-butylsulfonamido-1-(4-(4-2-pyrimidinyl)-1-pipérazinyl)-butyl),
41 - 1H-pyrazole-4-propylsulfonamido-1-(4-(4-(2-pyrimidinyl)-1-pipérazinyl)-butyl),
42 - 1H-pyrazole-4-éthylsulfonamido-1-(4-(4-(2-pyrimidinyl)-1-pipérazinyl)-butyl),
45 - 1H-pyrazole-4-sulfonique-1-(4-(4-(2-pyrimidinyl)-1-pipérazinyl)-butyl),
46 - 1H-imidazole-1-(4-(4-(2-pyrimidinyl)-1-pipérazinyl)-butyl),
47 - 1H-imidazole-2-méthyl-1-(4-(4-(2-pyrimidinyl)-1-pipérazinyl)-butyl),
48 - 1H-imidazole-4,5-dichloro-1-(4-(4-(2-pyrimidinyl)-1-pipérazinyl)-butyl),
49 - 1H-imidazole-4-méthyl-1-(4-(4-(2-pyrimidinyl)-1-pipérazinyl)-butyl),
50 - 1H-imidazole-5-méthyl-1-(4-(4-(2-pyrimidinyl)-1-pipérazinyl)-butyl),
51 - 1H-pyrazole-4-chloro-1-(4-(4-(2-pyrimidinyl)-1-pipérazinyl)-butyl) hydrochlorure,
52 - 1H-pyrazole-4-chloro-1-(4-(4-(2-pyrimidinyl)-1-pipérazinyl)-butyl) dihydrochlorure.

**3.** Composés hétérocycliques sélectionnés parmi le groupe suivant :

- 1H-pyrazole-4-benzamido-1-(4-(4-(2-pyrimidinyl)-1-pipérazinyl)-butyl),
- 1H-pyrazole-4-(4-méthoxyphényl)-1(4-(4-(2-pyrimidinyl)-1-pipérazinyl)-butyl),
- 1H-pyrazole-4-(4-chlorophényl)-1-(4-(4-(2-pyrimidinyl)-1-pipérazinyl)-butyl),
- 1H-pyrazole-4-phénylsulfonamido-1-(4-(4-(2-pyrimidyl)-1-pipérazinyl)-butyl),
- 1H-pyrazole-4-(4-méthylbenzene)sulfonamido-1-(4-(4-(2-pyrimidinyl)-1-pipérazinyl)-butyl),
- 1H-pyrazole-3,5-diméthyl-4-(N,N-diméthylsulfonamido)-1-(4-(4-(2-pyrimidinyl-1-pipérazinyl)-butyl),
- 1H-pyrazole-4-N-méthylsulfonamido-1(4-(4-(2-pyrimidinyl-1-pipérazinyl)-butyl),

**4.** Procédé de préparation des composés selon l'une des revendications 1 à 3, caractérisé par la mise en oeuvre d'au moins l'une des opérations suivantes :

4.1. Par réaction d'un composé de formule générale IV

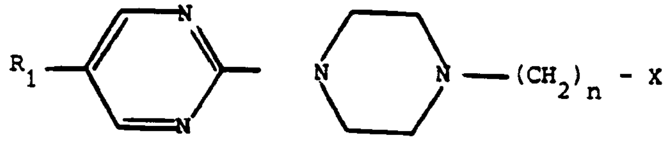

(IV)

dans laquelle

$R_1$ et n on les significations mentionnées précédemment et x représente un atome d'halogène, ou un groupe partant choisi parmi le mésyloxy ou le tosyloxy, avec un composé de formule V

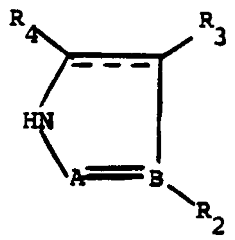

(V)

dans laquelle

A, B, $R_2$, $R_3$, $R_4$ et la liaison en pointillés, ont les significations mentionnées précédemment.

4.2. Par réaction d'un composé de formule générale VI

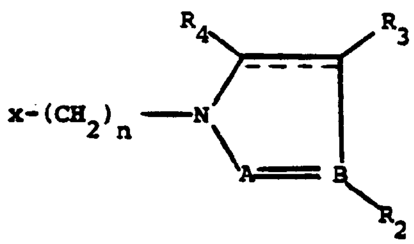

(VI)

dans laquelle

A, B, $R_2$, $R_3$, $R_4$, x et n ont les significations mentionnées précédemment, avec un composé de formule générale VII

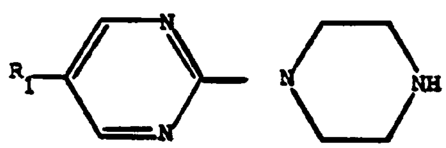

(VII)

dans laquelle

$R_1$ a les significations mentionnées précédemment.

4.3. Par réaction d'un composé de formule générale I dans laquelle $R_1$, $R_2$, $R_3$, $R_4$, Het et n ont les significations mentionnées précédemment, et dans laquelle au moins l'un des substituants $R_1$ ou $R_3$ représente un atome d'hydrogène, avec un halogène.

4.4. Par réaction d'un composé de formule générale I dans laquelle $R_1$, Het et n ont les significations mentionnées précédemment et $R_2$, $R_3$ ou $R_4$ représentent un groupe nitro, avec des agents réducteurs, pour obtenir un composé avec la même formule générale mais dans laquelle $R_2$, $R_3$ ou $R_4$ représente un groupe amino.

4.5. Par réaction d'un composé de formule générale I dans laquelle Het, $R_1$, l'un des substituants et n ont les significations mentionnées précédemment et $R_2$, $R_3$, n, $R_4$ représentent un groupe amino ou alkylamino, avec des anhydrides d'acides alkyle ou aryle carboxyliques, ou avec des halogénures d'acides aryle ou alkylsulfoniques.

**5.** A titre de médicaments, les dérivés selon les revendications 1 à 3, et leurs sels thérapeutiquement acceptables, en particulier à titre de médicaments destinés au traitement de certaines maladies du système nerveux central.

**6.** Compositions pharmaceutiques, caractérisées par le fait qu'elles contiennent, outre un support pharmaceutiquement acceptable, au moins un dérivé selon l'une des revendications 1 à 3, ou l'un de ses sels physiologiquement acceptables.

**7.** Utilisation des dérivés selon l'une des revendications 1 à 3, et leurs sels physiologiquement acceptables, pour la fabrication de médicaments destinés au traitement de l'anxiété, en particulier pour la fabrication de tranquilisants et/ou d'anxiolytiques.

**Patentansprüche**

1. Heterocyclische Verbindungen, dadurch gekennzeichnet, daß sie der allgemeinen Formel (I) entsprechen:

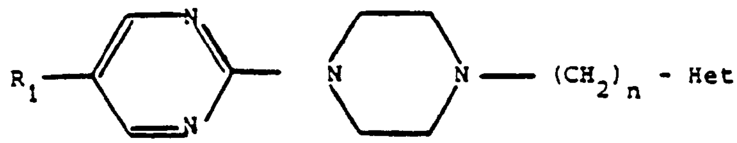

(I)

worin:

| | |
|---|---|
| $R_1$ | steht für ein Wasserstoffatom oder ein Halogenatom, |
| n | die Werte 1 bis 6 haben kann und |
| Het | steht für ein Azol oder eines seiner Derivate, ausgewählt aus der Grupe Imidazol, Indazol, den Tetrahydroindazolen, Pyrazol und Pyrazolin, das durch die allgemeine Formel (II) dargestellt werden kann: |

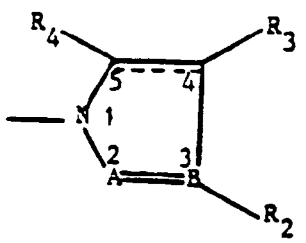

(II ) (II)

worin :

A und B, die immer verschieden sind, stehen für ein Kohlenstoffatom oder ein Stickstoffatom, die punktierte Linie eine gegebenenfalls vorhandene Doppelbindung zwischen den Positionen 4 und 5 anzeigt und

$R_2$, $R_3$ und $R_4$, die gleich oder verschieden sind, die auch einen Teil eines anderen aromatischen oder nicht-aromatischen Ringes bilden können, stehen für ein Wasserstoffatom, ein Halogenatom, einen niederen Alkylrest, einen Nitrorest, einen Hydroxyrest, einen Oxorest, einen Alkoxyrest, einen Cyanorest, einen Carboxylrest, einen Carboxamidorest, einen Alkylcarboxylatrest, einen Phenylrest, einen Sulfonrest, einen Sulfonamidorest, einen Aminorest oder einen substituierten Aminorest der allgemeinen Formel (III)

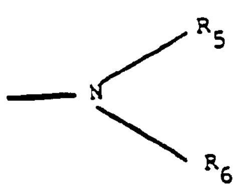

(III)

worin:

$R_5$ und $R_6$, die gleich oder verschieden sind, stehen für ein Wasserstoffatom, einen Alkylrest, einen Alkenylrest, einen Alkylcarboxyrest oder einen Alkylsulfonylrest.

2. Verbindungen der allgemeinen Formel (I) nach Anspruch 1, die ausgewählt werden aus der folgenden Gruppe:
   1 - 1H-Pyrazol -1-(4-(4-(2-pyrimidinyl)-1-piperazinyl)-butyl),
   2 - 1H-Pyrazol -3,5-dimethyl-1-(4-(4-(2-pyrimidinyl)-1-piperazinyl)-butyl),
   3 - 1H-Pyrazol -3,5-dimethyl-4-nitro-1-(4-(4-(2-pyrimidinyl)-1-piperazinyl-butyl),
   4 - 1H-Pyrazol -4-methyl-1-(4-(4-(2-pyrimidinyl)-1-piperazinyl)-butyl),
   5 - 1H-Indazol -1-(4-(4-(2-pyrimidinyl)-1-piperazinyl)-butyl),

6 - 1H-Pyrazol -3,5-dimethyl-4-bromo-1-(4-(4-(2-pyrimidinyl)-1-piperazinyl)-butyl),
7 - 1H-Pyrazol -4-nitro-1-(4-(4-(2-pyrimidinyl)-1-piperazinyl)-butyl),
8 - 1H-Pyrazol -4-chloro-1-(4-(4-(2-pyrimidinyl)-1-piperazinyl)-butyl),
9 - Ethyl -1-(4-(4-(2-pyrimidinyl)-1- piperazinyl)-butyl)-1H-pyrazol -4-carboxylat
10 - 1H-Pyrazolin -4-methyl-5-on -1-(4-(4-(2-pyrimidinyl)-1-piperazinyl)-butyl)
11 - 1H-Pyrazol -3-methyl-5-phenyl-1-(4-(4-(2-pyrimidinyl)-1-piperazinyl)-butyl),
12 - 1H-Pyrazol -4-bromo-1-(4-(4-(2-pyrimidinyl)-1-piperazinyl)-butyl),
13 - 1H-Pyrazol -4-cyano-1-(4-(4-(2-pyrimidinyl)-1-piperazinyl)-butyl),
14 - 1H-Pyrazol -4-fluoro-1-(4-(4-(2-pyrimidinyl)-1-piperazinyl)-butyl),
15 - 1H-Pyrazol -4-methoxy-1-(4-(4-(2-pyrimidinyl)-1-piperazinyl)-butyl),
16 - 1H-Pyrazol -4-amino-1-(4-(4-(2-pyrimidinyl)-1-piperazinyl)-butyl),
17 - 1H-Pyrazol -4-methylsulfonamido-1-(4-(4-(2-pyrimidinyl)-1-piperazinyl)-butyl),
19 - 1H-Pyrazol -4-acetamido-1-(4-(4-(2-pyrimidinyl)-1-piperazinyl)-butyl),
20 - 1H-Pyrazol -4-(2-butyl)amino-1-(4-(4-(2-pyrimidinyl)-1-piperazinyl)-butyl),
21 - 1H-Pyrazol -4-bromo-1-(4-(4-(5-bromopyrimidin-2-yl)-1-piperazinyl)-butyl),
22 - 1H-Pyrazol -4-bromo-1-(4-(4-(5-chloropyrimidin-2-yl)-1-piperazinyl)-butyl),
23 - 1H-Pyrazol -5-methyl-1-(4-(4-(2-pyrimidinyl)-1-piperazinyl)-butyl),
24 - 1H-Pyrazol -3-methyl-1-(4-(4-(2-pyrimidinyl)-1-piperazinyl)-butyl),
25 - 1H-Pyrazol -4-bromo-5-méthyl-1-(4-(4-(2-pyrimidinyl)-1-piperazinyl)-butyl),
26 - 1H-Pyrazol -4-bromo-3-methyl-1-(4-(4-(2-pyrimidinyl)-1-piperazinyl)-butyl)
27 - 1H-4,5,6,7-Tetrahydroindazol -1-(4-(4-(2-pyrimidinyl)-1-piperazinyl)-butyl),
28 - 2H-3,4,5,6-Tetrahydroindazol -2-(4-(4-(2-pyrimidinyl)-1-piperazinyl)-butyl),
29 - 1H-Pyrazol -5-methyl-4-phenyl-1-(4-(4-(2-pyrimidinyl)-1-piperazinyl)-butyl),
30 - 1H-Pyrazol -3-methyl-4-phenyl-1-(4-(4-(2-pyrimidinyl)-1-piperazinyl)-butyl),
31 - 1H-Pyrazol -3-chloro-4-fluoro-1-(4-(4-(2-pyrimidinyl)-1-piperazinyl)-butyl),
32 - 1H-Pyrazol -3-chloro-4-methoxy-1-(4-(4-(2-pyrimidinyl)-1-piperazinyl)-butyl),
36 - 1H-Pyrazol -4-phenyl-1-(4-(4-(2-pyrimidinyl)-1-piperazinyl)-butyl),
37 - 1H-Pyrazol -3,5-diphenyl-1-(4-(4-(2-pyrimidinyl)-1-piperazinyl)-butyl),
40 - 1H-Pyrazol -4-butylsulfonamido-1-(4-(4-2-pyrimidinyl)-1-pipérazinyl)-butyl),
41 - 1H-Pyrazol -4-propylsulfonamido-1-(4-(4-(2-pyrimidinyl)-1-piperazinyl)-butyl),
42 - 1H-Pyrazol -4-ethylsulfonamido-1-(4-(4-(2-pyrimidinyl)-1-piperazinyl)-butyl),
45 - 1H-Pyrazol -4-sulfon -1-(4-(4-(2-pyrimidinyl)-1-piperazinyl)-butyl),
46 - 1H-Imidazol -1-(4-(4-(2-pyrimidinyl)-1-piperazinyl)-butyl),
47 - 1H-Imidazol -2-methyl-1-(4-(4-(2-pyrimidinyl)-1-piperazinyl)-butyl),
48 - 1H-Imidazol -4,5-dichloro-1-(4-(4-(2-pyrimidinyl)-1-piperazinyl)-butyl),
49 - 1H-Imidazol -4-methyl-1-(4-(4-(2-pyrimidinyl)-1-piperazinyl)-butyl),
50 - 1H-Imidazol -5-methyl-1-(4-(4-(2-pyrimidinyl)-1-piperazinyl)-butyl),
51 - 1H-Pyrazol -4-chloro-1-(4-(4-(2-pyrimidinyl)-1-piperazinyl)-butyl) hydrochlorid,
52 - 1H-Pyrazol -4-chloro-1-(4-(4-(2-pyrimidinyl)-1-piperazinyl)-butyl) dihydrochlorid.

**3.** Heterocyclische Verbindungen, die ausgewählt werden aus der folgenden Gruppe:
- 1H-Pyrazol -4-benzamido-1-(4-(4-(2-pyrimidinyl)-1-piperazinyl)-butyl),
- 1H-Pyrazol -4-(4-methoxyphenyl)-1(4-(4-(2-pyrimidinyl)-1-piperazinyl)-butyl),
- 1H-Pyrazol -4-(4-chlorophenyl)-1-(4-(4-(2-pyrimidinyl)-1-piperazinyl)-butyl),
- 1H-Pyrazol -4-phenylsulfonamido-1-(4-(4-(2-pyrimidyl)-1-piperazinyl)-butyl),
- 1H-Pyrazol -4-(4-methylbenzene)sulfonamido-1-(4-(4-(2-pyrimidinyl)-1-piperazinyl)-butyl),
- 1H-Pyrazol -3,5-dimethyl-4-(N,N-dimethylsulfonamido)-1-(4-(4-(2-pyrimidinyl-1-piperazinyl)-butyl),
- 1H-Pyrazol -4-N-methylsulfonamido-1(4-(4-(2-pyrimidinyl-1-piperazinyl)-butyl),

**4.** Verfahren zur Herstellung der Verbindungen nach einem der Ansprüche 1 bis 3, dadurch gekennzeichnet, daß man mindestens eine der folgenden Operationen durchfürt:
- 4.1. Umsetzung einer Verbindung der allgemeinen Formel (IV)

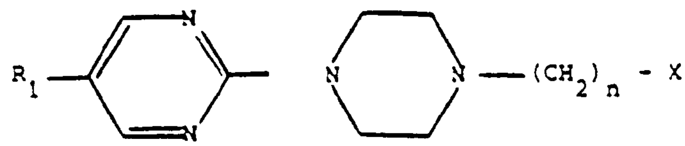

(IV)

worin $R_1$ und n die oben angegebenen Bedeutungen haben und X steht für ein Halogenatom oder eine austretende Gruppe, ausgewählt aus Mesyloxy oder Tosyloxy, mit einer Verbindung der Formel (V)

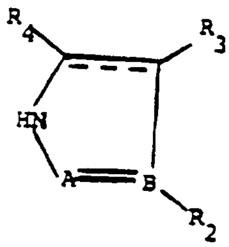

(V)

worin A, B, $R_2$, $R_3$, $R_4$ und die punktierte Bindung die obengenannten Bedeutungen haben;

- 4.2. Umsetzung einer Verbindung der allgemeinen Formel (VI)

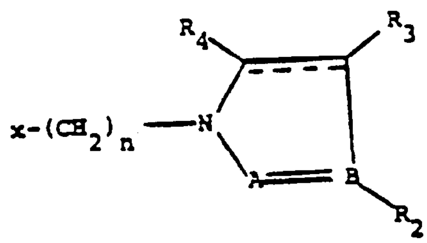

(VI)

worin A, B, $R_2$, $R_3$, $R_4$, x und n die oben angegebenen Bedeutungen haben,
mit einer Verbindung der allgemeinen Formel (VII)

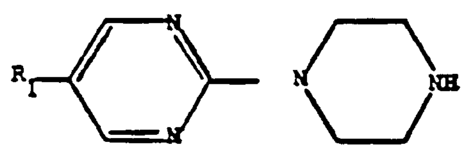

(VII)

worin
$R_1$ die oben angegebenen Bedeutungen hat;

- 4.3. Umsetzung einer Verbindung der allgemeinen Formel (I), in der $R_1$, $R_2$, $R_3$, $R_4$, Het und n die oben angegebenen Bedeutungen haben und in der mindestens einer der Substituenten $R_1$ oder $R_3$ ein Wasserstoffatom darstellt, mit einem Halogen;
- 4.4. Umsetzung einer Verbindung der allgemeinen Formel (I), in der $R_1$, Het und n die oben angegebenen Bedeutungen haben und $R_2$, $R_3$ oder $R_4$ eine Nitrogruppe darstellt, mit Reduktionsmitteln unter Bildung einer Verbindung mit der gleichen allgemeinen Formel, in der jedoch $R_2$, $R_3$ oder $R_4$ eine Aminogruppe darstellt;
- 4.5. Umsetzung einer Verbindung der allgemeinen Formel (I), in der Het, $R_1$, einer der Substituenten und n die oben angegebenen Bedeutungen haben und $R_2$, $R_3$, n, $R_4$ stehen für eine Aminogruppe oder eine Alkylaminogruppe, mit Alkyl- oder Arylcarbonsäureanhydriden oder mit Aryl- oder Alkylsulfonsäurehalogeniden.

5. Als Arzneimittel die Derivate nach den Ansprüchen 1 bis 3 und ihre therapeutisch akzeptablen Salze, insbesondere als Arzneimittel für die Behandlung bestimmter Erkrankungen des Zentralnervensystems.

6. Pharmazeutische Zusammensetzungen, dadurch gekennzeichnet, daß sie außer einem pharmazeutisch akzeptablen Träger mindestens ein Derivat nach einem der Ansprüche 1 bis 3 oder eines seiner physiologisch akzeptablen Salze enthalten.

7. Verwendung der Derivate nach einem der Asprüche 1 bis 3 und ihrer physiologisch akzeptablen Salze für die Herstellung von Arzneimitteln für die Behandlung von Angstzuständen, insbesondere für die Herstellung von Tranquilizern und/oder Anxiolytika.

## Claims

1. Heterocyclic compounds characterized in that they correspond to the general formula I:

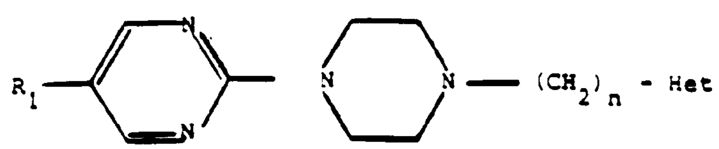

I

in which $R_1$ represents a hydrogen atom or a halogen, n can have the values 1 to 6 and Het represents an azole, or one of its derivatives selected from among imidazole, indazole, tetrahydroindazoles, pyrazole and pyrazoline and which can be represented by the general formula II:

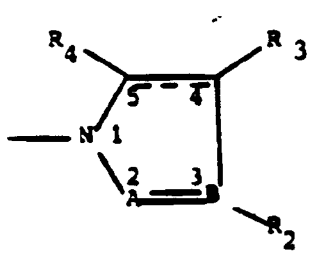

II

in which A and B, always different, represent a carbon atom or a nitrogen atom, the dashed line indicates the possible presence of a double bond between positions 4 and 5 and $R_2$, $R_3$ and $R_4$, identical or different, and which can also from a part of another ring, aromatic or not, represent a hydrogen atom, a halogen, a lower alkyl radical, a nitro radical, a hydroxy radical, an oxo radical, an alkoxy radical, a cyano radical, a carboxylic radical, a carboxamido radical, an alkyl carboxylate radical, a phenyl radical, a sulfonic radical, a sulfonamido radical, an amino or substituted amino radical, of general formula III:

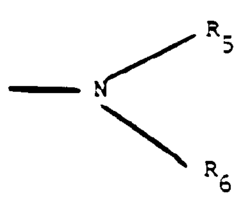

III

in which $R_5$ and $R_5$, identical or different, represent a hydrogen atom, an alkyl radical, an alkenyl radical, an alkylcarboxy radical, an alkylsulfonyl radical.

2. The compounds corresponding to the general formula I according to claim 1, selected from among the following goup:
   1 - 1H-pyrazole-1-(4-(4-(2-pyrimidinyl)-1-pipérazinyl)-butyl),
   2 - 1H-pyrazole-3,5-diméthyl-1-(4-(4-(2-pyrimidinyl)-1-pipérazinyl)-butyl),
   3 - 1H-pyrazole-3,5-diméthyl-4-nitro-1-(4-(4-(2-pyrimidinyl)-1-pipérazinyl-butyl),

4 - 1H-pyrazole-4-méthyl-1-(4-(4-(2-pyrimidinyl)-1-pipérazinyl)-butyl),
5 - 1H-indazole-1-(4-(4-(2-pyrimidinyl)-1-pipérazinyl)-butyl),
6 - 1H-pyrazole-3,5-diméthyl-4-bromo-1-(4-(4-(2-pyrimidinyl)-1-pipérazinyl)-butyl),
7 - 1H-pyrazole-4-nitro-1-(4-(4-(2-pyrimidinyl)-1-pipérazinyl)-butyl),
8 - 1H-pyrazole-4-chloro-1-(4-(4-(2-pyrimidinyl)-1-pipérazinyl)-butyl),
9 - 1H-pyrazole-4-ethylcarboxylate-1-(4-(4-(2-pyrimidinyl)-1-pipérazinyl)-butyl),
10 - 1H-pyrazoline-4-méthyl-5-one-1-(4-(4-(2-pyrimidinyl)-1-pipérazinyl)-butyl)
11 - 1H-pyrazole-3-méthyl-5-phényl-1-(4-(4-(2-pyrimidinyl)-1-pipérazinyl)-butyl),
12 - 1H-pyrazole-4-bromo-1-(4-(4-(2-pyrimidinyl)-1-pipérazinyl)-butyl),
13 - 1H-pyrazole-4-cyano-1-(4-(4-(2-pyrimidinyl)-1-pipérazinyl)-butyl),
14 - 1H-pyrazole-4-fluoro-1-(4-(4-(2-pyrimidinyl)-1-pipérazinyl)-butyl),
15 - 1H-pyrazole-4-méthoxy-1-(4-(4-(2-pyrimidinyl)-1-pipérazinyl)-butyl),
16 - 1H-pyrazole-4-amino-1-(4-(4-(2-pyrimidinyl)-1-pipérazinyl)-butyl),
17 - 1H-pyrazole-4-méthylsulfonamido-1-(4-(4-(2-pyrimidinyl)-1-pipérazinyl)-butyl),
19 - 1H-pyrazole-4-acétamido-1-(4-(4-(2-pyrimidinyl)-1-pipérazinyl)-butyl),
20 - 1H-pyrazole-4-(2-butyl)amino-1-(4-(4-(2-pyrimidinyl)-1-pipérazinyl)-butyl),
21 - 1H-pyrazole-4-bromo-1-(4-(4-(5-bromopyrimidin-2-yl)-1-pipérazinyl)-butyl),
22 - 1H-pyrazole-4-bromo-1-(4-(4-(5-chloropyrimidin-2-yl)-1-pipérazinyl)-butyl),
23 - 1H-pyrazole-5-méthyl-1-(4-(4-(2-pyrimidinyl)-1-pipérazinyl)-butyl),
24 - 1H-pyrazole-3-méthyl-1-(4-(4-(2-pyrimidinyl)-1-pipérazinyl)-butyl),
25 - 1H-pyrazole-4-bromo-5-méthyl-1-(4-(4-(2-pyrimidinyl)-1-pipérazinyl)-butyl),
26 - 1H-pyrazole-4-bromo-3-méthyl-1-(4-(4-(2-pyrimidinyl)-1-pipérazinyl)-butyl)
27 - 1H-4,5,6,7-tétrahydroindazole-1-(4-(4-(2-pyrimidinyl)-1-pipérazinyl)-butyl),
28 - 2H-3,4,5,6-tétrahydroindazole-2-(4-(4-(2-pyrimidinyl)-1-pipérazinyl)-butyl),
29 - 1H-pyrazole-5-méthyl-4-phényl-1-(4-(4-(2-pyrimidinyl)-1-pipérazinyl)-butyl),
30 - 1H-pyrazole-3-méthyl-4-phényl-1-(4-(4-(2-pyrimidinyl)-1-pipérazinyl)-butyl),
31 - 1H-pyrazole-3-chloro-4-fluoro-1-(4-(4-(2-pyrimidinyl)-1-pipérazinyl)-butyl),
32 - 1H-pyrazole-3-chloro-4-méthoxy-1-(4-(4-(2-pyrimidinyl)-1-pipérazinyl)-butyl),
40 - 1H-pyrazole-4-butylsulfonamido-1-(4-(4-2-pyrimidinyl)-1-pipérazinyl)-butyl),
41 - 1H-pyrazole-4-propylsulfonamido-1-(4-(4-(2-pyrimidinyl)-1-pipérazinyl)-butyl),
42 - 1H-pyrazole-4-ethylsulfonamido-1-(4-(4-(2-pyrimidinyl)-1-pipérazinyl)-butyl),
45 - 1H-pyrazole-4-sulfonique-1-(4-(4-(2-pyrimidinyl)-1-pipérazinyl)-butyl),
46 - 1H-imidazole-1-(4-(4-(2-pyrimidinyl)-1-pipérazinyl)-butyl),
47 - 1H-imidazole-2-méthyl-1-(4-(4-(2-pyrimidinyl)-1-pipérazinyl)-butyl),
48 - 1H-imidazole-4,5-dichloro-1-(4-(4-(2-pyrimidinyl)-1-pipérazinyl)-butyl),
49 - 1H-imidazole-4-méthyl-1-(4-(4-(2-pyrimidinyl)-1-pipérazinyl)-butyl),
50 - 1H-imidazole-5-méthyl-1-(4-(4-(2-pyrimidinyl)-1-pipérazinyl)-butyl),
51 - 1H-pyrazole-4-chloro-1-(4-(4-(2-pyrimidinyl)-1-pipérazinyl)-butyl) hydrochlorure,
52 - 1H-pyrazole-4-chloro-1-(4-(4-(2-pyrimidinyl)-1-pipérazinyl)-butyl) dihydrochlorure.

**3.** Heterocyclic compound selected from among the following groups:
- 1H-pyrazole-4-benzamido-1-(4-(4-(2-pyrimidinyl)-1-pipérazinyl)-butyl),
- 1H-pyrazole-4-(4-méthoxyphényl)-1(4-(4-(2-pyrimidinyl)-1-pipérazinyl)-butyl),
- 1H-pyrazole-4-(4-chlorophényl)-1-(4-(4-(2-pyrimidinyl)-1-pipérazinyl)-butyl),
- 1H-pyrazole-4-phénylsulfonamido-1-(4-(4-(2-pyrimidyl)-1-pipérazinyl)-butyl),
- 1H-pyrazole-4-(4-méthylbenzene)sulfonamido-1-(4-(4-(2-pyrimidinyl)-1-pipérazinyl)-butyl),
- 1H-pyrazole-3,5-diméthyl-4-(N,N-diméthylsulfonamido)-1-(4-(4-(2-pyrimidinyl-1-pipérazinyl)-butyl),
- 1H-pyrazole-4-N-méthylsulfonamido-1(4-(4-(2-pyrimidinyl-1-pipérazinyl)-butyl),

**4.** Process for the preparation of compounds according to one of claims 1 to 3, characterized by the employment of at least one of the following operations:

4.1 By reaction of a compound of the general formula IV :

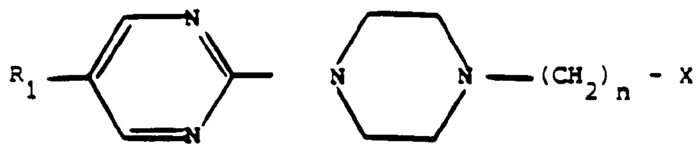

IV

in which $R_1$ and n have the previously mentioned meanings and x represents a halogen atom, or a detachable group selected from among mesyloxy or tosyloxy, with a compound of the general formula V:

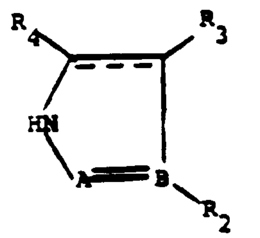

V

in which A, B, $R_2$, $R_3$, $R_4$ and the bond in dashed line, have the previously mentioned meanings.

4.2 By reaction of a compound of the general formula VI:

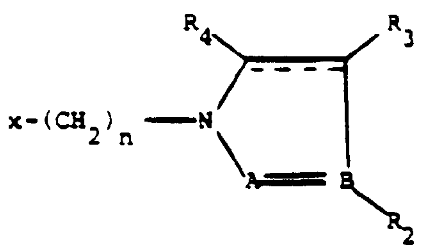

VI

in which A, B, $R_2$, $R_3$, $R_4$, x and n have the previously mentioned meanings, with a compound of the general formula VII:

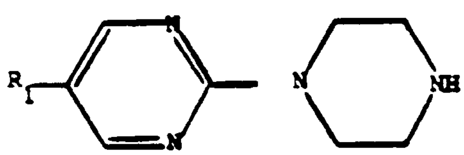

VII

in which $R_1$ has the previously mentioned meanings.

4.3 By reaction of a compound of the general formula I in which $R_1$, $R_2$, $R_3$, $R_4$ Het and n have the previously mentioned meanings, and in which at least one of the substituents $R_1$ of $R_3$, represents a hydrogen atom, with a halogen.

4.4 By reaction of a compound of the general I in which $R_1$, Het and n have the previously mentioned meanings and $R_2$, $R_3$ or $R_4$ represent a nitro group, with reducing agents, to obtain a compound with the same general formula but in which $R_2$, $R_3$ or $R_4$ represent an amino group.

4.5 By reaction of a compound of the general formula I in which Het, $R_1$, one of the substituents and n have the previously mentioned meanings and $R_2$, $R_3$, n and $R_4$ represent an amino or alkylamino group, with anhydrides of alkyl or aryl carboxylic acids, or with halogenides of aryl or alkylsulfonic acids.

5. As medicaments, the derivatives of the general formula I and their therapeutically acceptable salts, according to claims 1 to 3, in particular as medicaments intended for the treatment of certain disorders of the central nervous system.

6. Pharmaceutical compositions, characterized by the fact that they contain, besides a pharmaceutically acceptable support, at least one derivative of the general formula I or one of its physiologically acceptable salts, according to one of claims 1 to 3.

**7.** Use of derivatives of the general formula I and their physiologically acceptable salts, according to one of claims 1 to 3, for the manufacture of medicaments intended for the treatment of anxiety, in particular for the manufacture of tranquilizers and/or anxiolytic agents.